# EUROPEAN PATENT APPLICATION

(11) **EP 4 056 532 A1**
(43) Date of publication of application: **14.09.2022**
(21) Application number: 21020142.2
(22) Date of filing: 12.03.2021
(51) Int. Cl.: C01B 3/38, C07C 29/151

(54) **METHOD AND APPARATUS FOR REFORMING HYDROCARBON GAS FOR METHANOL PRODUCTION**

(71) Applicant: L'Air Liquide Société Anonyme pour l'Etude et l'Exploitation des Procédés Georges Claude, 75321 Paris (FR)
(72) Inventor: Kopetsch, Hans, D-60439 Frankfurt am Main (DE); Williams, Bryce, D-60439 Frankfurt am Main (DE); Covella, Karsten, D-60388 Frankfurt am Main (DE); Aoun, Julien, D-60439 Frankfurt am Main (DE)
(74) Representative: Dropsch, Holger

(57) **Abstract**

A method for producing a synthesis gas for methanol synthesis is provided. The method includes mixing a first portion of hydrocarbon gas with steam to produce a first hydrocarbon gas-steam mixture; pre-heating the first hydrocarbon gas-steam mixture before supplying the first hydrocarbon gas-steam mixture with an oxidant gas comprising molecular oxygen into a partial oxidation chamber to produce a synthesis gas effluent; mixing a second portion of the hydrocarbon gas with steam to produce a second hydrocarbon gas-steam mixture which is preheated before combining with the synthesis gas effluent; combining the synthesis gas effluent with the second hydrocarbon gas-steam mixture to produce a synthesis gas mixture; and producing a reformed synthesis gas by adiabatically reforming the synthesis gas mixture over at least one reforming catalyst in a reforming chamber (108, 208) to convert the second portion of hydrocarbon gas from the second hydrocarbon gas-steam mixture into the synthesis gas.

## Description

### TECHNICAL FIELD

The present disclosure relates generally to reforming hydrocarbon gas; more specifically, the present disclosure relates to a method and apparatus for reforming hydrocarbon gas for producing methanol.

### BACKGROUND

Conventionally, methanol is produced on an industrial scale from synthesis gas, namely "syngas," which is a combination of varying amounts of hydrogen (H₂), carbon monoxide (CO), and carbon dioxide (CO₂). The most common process for producing a methanol synthesis gas from hydrocarbon gas is catalytic steam reforming using up to three catalytic steps for large production capacities that includes pre-reforming, primary reforming, and secondary reforming for an optimum synthesis gas composition. For small production capacities, a single reforming step is applied, for example steam methane reforming (SMR), resulting in suitable synthesis gas but contains too much hydrogen. With reference to the Stoichiometric Number (SN), such a gas is often referred to as being over-stoichiometric. Among the known synthesis gas generation processes, Partial Oxidation (POX) exhibits the lowest steam-to-carbon ratios, down to about 0.1 or lower (for example to zero), and high process temperatures of up to about 1400 °C. Such POX allows small Partial Oxidation (POX) reactor sizes to be used and the highest conversion of hydrocarbons to CO and CO₂. The resulting stoichiometric number SN = (H₂ - CO₂) / (CO + CO₂) of the synthesis gas is usually less than about 1.7 and hence unsuitable for direct use as a methanol synthesis make-up gas.

Single stage steam reforming is a well-proven process for generating methanol synthesis gas; however, the steam reformer is a rather expensive equipment and difficult to operate. Conversely, reforming by catalytic partial oxidation, and even more by non-catalytic partial oxidation, yield clearly sub-stoichiometric synthesis gases, which are unsuitable for direct use in methanol synthesis. The sub-stoichiometric synthesis gases do not contain sufficient amounts of hydrogen. Even adding all hydrogen recovered from the purge gas of methanol synthesis unit (by pressure swing adsorption, membrane, or other techniques) to the synthesis gas is not sufficient. This deficit can be overcome by reclaiming hydrogen from part of the synthesis gas itself in existing systems. However, this results in a higher overall gas usage per unit methanol produced.

Therefore, there is a need to address the aforementioned technical drawbacks in existing technologies in producing a synthesis gas for methanol synthesis.

### SUMMARY

The present disclosure seeks to provide a more efficient method and apparatus for a combined catalytic and non-catalytic reforming of hydrocarbon gas to produce a synthesis gas with a higher stoichiometric number SN = (H₂ - CO₂) / (CO + CO₂) with molar fractions of hydrogen (H₂), carbon monoxide (CO) and carbon dioxide (CO₂) which is more suitable for downstream methanol synthesis. An aim of the present disclosure is to provide a solution that overcomes, at least partially, the problems encountered in prior art and provide an improved method and apparatus for producing stoichiometric methanol synthesis gas by performing a non-catalytic partial oxidation of hydrocarbon gas followed by an adiabatic catalytic reforming step and routing a portion of the hydrocarbon gas directly to the adiabatic catalytic reforming step. The object of the present disclosure is achieved by the solutions provided in the enclosed independent claims. Advantageous implementations of the present disclosure are further defined in the dependent claims.

An adiabatic catalytic reforming step is to be understood to use a catalytic bed that is maintained under conditions where the reforming of hydrocarbons with steam progresses to produce synthesis gas; moreover, there is no heat addition or removal from external sources. Typically, this reforming step results in a hot feedstock that cools as endothermic reforming reactions of the reforming step progress.

According to a first aspect, the present disclosure provides a method for producing a synthesis gas for methanol synthesis, the method comprising: mixing a first portion of hydrocarbon gas with steam to produce a first hydrocarbon gas-steam mixture; pre-heating the first hydrocarbon gas-steam mixture before supplying the first hydrocarbon gas-steam mixture with an oxidant gas comprising molecular oxygen into a partial oxidation chamber to produce a synthesis gas effluent; mixing a second portion of the hydrocarbon gas with steam to produce a second hydrocarbon gas-steam mixture, wherein the second hydrocarbon gas-steam mixture is pre-heated before combining with the synthesis gas effluent; combining the synthesis gas effluent with the second hydrocarbon gas-steam mixture to produce a synthesis gas mixture; and producing a reformed synthesis gas by adiabatically reforming the synthesis gas mixture over at least one reforming catalyst in an adiabatic reforming chamber.

The method of producing the synthesis gas for methanol synthesis according to present disclosure is of advantage on that the method enables the production of stoichiometric synthesis gas from the hydrocarbon gas; wherein the production occurs with low operational costs. The method enables the production of the synthesis gas with a higher SN by a combined non-catalytic partial oxidation and adiabatic catalytic reforming of hydrocarbon gas which is more suitable for downstream methanol synthesis than currently existing technologies. Reformed synthesis gas produced by the method has an SN of about 1.8. Optionally, "about" here means +/- 30% from nominal value.

According to a second aspect, the present disclosure provides a method for producing methanol from hydrocarbon gas, the method comprising:
(i) mixing a first portion of the hydrocarbon gas with steam to produce a first hydrocarbon gas-steam mixture;
(ii) pre-heating the first hydrocarbon gas-steam mixture before supplying the first hydrocarbon gas-steam mixture with an oxidant gas comprising molecular oxygen into a partial oxidation chamber to produce a synthesis gas effluent;
(iii) mixing a second portion of the hydrocarbon gas with steam to produce a second hydrocarbon gas-steam mixture, wherein the second hydrocarbon gas-steam mixture is pre-heated before combining with the synthesis gas effluent;
(iv) combining the synthesis gas effluent with the second hydrocarbon gas-steam mixture to produce a synthesis gas mixture;
(v) producing a reformed synthesis gas by adiabatically reforming the synthesis gas mixture over at least one reforming catalyst in an adiabatic reforming chamber;
(vi) combining the reformed synthesis gas with recovered hydrogen to form a methanol synthesis make-up gas;
(vii) adding the methanol synthesis make-up gas to a methanol synthesis loop;
(viii) reacting the methanol synthesis make-up gas in a methanol synthesis unit to produce a crude methanol stream and a purge gas stream;
(ix) processing the purge gas stream in a hydrogen recovery unit to recover hydrogen from the purge gas stream; and
(x) returning the recovered hydrogen to the methanol synthesis loop via the methanol synthesis make-up gas.

The method for producing methanol from hydrocarbon gas according to present disclosure is of advantage in that the method enables a more efficient production of methanol which utilizes the synthesis gas with a stoichiometric number higher than that of pure partial oxidation (POX) gas. The reformed synthesis gas produced by the method has an SN of about 1.8 and the methanol synthesis make-up gas has an SN of about 2.0. Optionally, "about" here means +/- 30% from nominal value. The method includes a 2-stage synthesis gas production process that takes advantage of a combined non-catalytic partial oxidation and catalytic adiabatic reforming of hydrocarbon gas for producing a synthesis gas suitable for methanol production. An additional advantage of the method according to the present disclosure is that the method does not require hydrogen recovery from the reformed synthesis gas; instead, the hydrogen is recovered only from the purge gas. The method enables the production of synthesis gas with a higher SN which is more suitable for downstream methanol synthesis.

According to a third aspect, the present disclosure provides an apparatus for producing a synthesis gas for methanol synthesis, the apparatus comprising: a hydrocarbon gas feed supply system comprising,
a first line for delivering a first portion of the hydrocarbon gas; and
a second line for delivering a second portion of the hydrocarbon gas,
wherein the first portion of the hydrocarbon gas and the second portion of the hydrocarbon gas are mixed with steam to produce a first hydrocarbon gas-steam mixture and a second hydrocarbon gas-steam mixture respectively;
a partial oxidation chamber comprising
an inlet for receiving the first hydrocarbon gas-steam mixture and means for mixing the first hydrocarbon gas-steam mixture with an oxidant gas comprising molecular oxygen from an oxidant gas supply system to produce a synthesis gas effluent, wherein the partial oxidation chamber performs partial oxidation on the first hydrocarbon gas-steam mixture using the oxidant gas comprising molecular oxygen to produce the synthesis gas effluent;
and an outlet for providing the synthesis gas effluent to combine with the second hydrocarbon gas-steam mixture, wherein the synthesis gas effluent is combined with the second hydrocarbon gas-steam mixture to produce a synthesis gas mixture;
an adiabatic reforming chamber that is located downstream to the partial oxidation chamber for producing a reformed synthesis gas by adiabatically reforming the synthesis gas mixture received from the partial oxidation chamber, over at least one reforming catalyst in the adiabatic reforming chamber.

The apparatus for producing the synthesis gas for methanol synthesis according to present disclosure enables the production of stoichiometric synthesis gas from the hydrocarbon gas with lower operational costs. The apparatus enables the production of synthesis gas with a higher SN by performing combined non-catalytic partial oxidation (POX) and catalytic reforming of hydrocarbon gas which is more suitable for downstream methanol synthesis. The reformed synthesis gas produced by the apparatus has an SN of about 1.8. Optionally, "about" here means +/- 30% from nominal value.

According to a fourth aspect, the present disclosure provides an apparatus for producing a methanol from hydrocarbon gas, the apparatus comprising: a hydrocarbon gas feed chamber comprising
a first line for delivering a first portion of the hydrocarbon gas; and
a second line for delivering a second portion of the hydrocarbon gas, wherein the first portion of the hydrocarbon gas and the second portion of the hydrocarbon gas are mixed with steam to produce a first hydrocarbon gas-steam mixture and a second hydrocarbon gas-steam mixture respectively;
a partial oxidation chamber comprising
a first inlet for receiving the first hydrocarbon gas-steam mixture and means for mixing the first hydrocarbon gas-steam mixture with an oxidant gas comprising molecular oxygen from an oxidant gas supply system to produce a synthesis gas effluent, wherein the partial oxidation chamber performs partial oxidation on the first hydrocarbon gas-steam mixture using the oxidant gas comprising molecular oxygen to produce the synthesis gas effluent; and
a first outlet for providing the synthesis gas effluent to combine with the second hydrocarbon gas-steam mixture, wherein the synthesis gas effluent is combined with the second hydrocarbon gas-steam mixture to produce a synthesis gas mixture;
an adiabatic reforming chamber that is located downstream to the partial oxidation chamber for producing a reformed synthesis gas by adiabatically reforming the synthesis gas mixture, received from the partial oxidation chamber, over at least one reforming catalyst in the adiabatic reforming chamber;
a methanol synthesis unit for converting the synthesis gas into a crude methanol stream and a purge gas stream;
a hydrogen recovery unit for recovering hydrogen from the purge gas stream, comprising an inlet for receiving the purge gas stream and two outlets for delivering respectively the recovered hydrogen and a tail gas, wherein the recovered hydrogen is combined with the reformed synthesis gas to produce a methanol synthesis make-up gas, wherein the methanol synthesis make-up gas is provided into the methanol synthesis unit to produce the crude methanol stream and the purge gas stream.

The apparatus for producing a methanol from hydrocarbon gas according to the present disclosure is of advantage in that the apparatus enables a more efficient production of methanol which utilizes the synthesis gas with a stoichiometric number higher than that of pure partial oxidation (POX) gas. The reformed synthesis gas produced by the apparatus has an SN of about 1.8 and the methanol synthesis make-up gas has an SN of about 2.0. Optionally, "about" here means +/- 30% from nominal value. The apparatus facilitates a 2-stage synthesis gas production process that takes advantage of a combined non-catalytic partial oxidation and adiabatic catalytic reforming of hydrocarbon gas for producing the methanol. The apparatus is of advantage in that the apparatus enables the production of synthesis gas with a higher SN which is more suitable for downstream methanol synthesis at low operational costs. The apparatus advantageously benefits from using a catalytic reformer that provides a simple and straightforward process layout for the production of the synthesis gas. Construction costs of the apparatus are reduced because of its compact design which can easily be achieved in a single-train.

Embodiments of the present disclosure eliminate the aforementioned drawbacks in existing known approaches for reforming hydrocarbon gas for the methanol production. The advantage of the embodiments according to the present disclosure is that the embodiments enable the production of synthesis gas with a higher SN which is more suitable for downstream methanol synthesis with minimal capital investment and low operational costs.

Additional aspects, advantages, features and objects of the present disclosure are made apparent from the drawings and the detailed description of the illustrative embodiments construed in conjunction with the appended claims that follow. It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. To illustrate the present disclosure, exemplary constructions of the disclosure are shown in the drawings. However, the present disclosure is not limited to specific methods and instrumentalities disclosed herein. Moreover, those in the art will understand that the drawings are not to scale. Wherever possible, the same elements have been indicated by identical numbers. Embodiments of the present disclosure will now be described, by way of example only, with reference to the following diagrams wherein:
FIG. 1 is a schematic illustration of an apparatus for producing a synthesis gas for methanol synthesis according to an embodiment of the present disclosure;
FIG. 2 is a schematic illustration of an apparatus for producing a methanol from hydrocarbon gas according to an embodiment of the present disclosure;
FIG. 3 is a flowchart illustrating a method for producing a synthesis gas for methanol synthesis according to an embodiment of the present disclosure; and
FIGS. 4A and 4B are flowcharts illustrating a method for producing a methanol from hydrocarbon gas according to an embodiment of the present disclosure.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

According to a first aspect, there is provided a method for producing a synthesis gas for methanol synthesis, the method comprising: mixing a first portion of hydrocarbon gas with steam to produce a first hydrocarbon gas-steam mixture; pre-heating the first hydrocarbon gas-steam mixture before supplying the first hydrocarbon gas-steam mixture with an oxidant gas comprising molecular oxygen into a partial oxidation chamber to produce a synthesis gas effluent; mixing a second portion of the hydrocarbon gas with steam to produce a second hydrocarbon gas-steam mixture, wherein the second hydrocarbon gas-steam mixture is pre-heated before combining with the synthesis gas effluent; combining the synthesis gas effluent with the second hydrocarbon gas-steam mixture to produce a synthesis gas mixture; and producing a reformed synthesis gas by adiabatically reforming the synthesis gas mixture over at least one reforming catalyst in an adiabatic reforming chamber.

The method of producing the synthesis gas for methanol synthesis according to present disclosure enables the production of stoichiometric synthesis gas by performing a non-catalytic partial oxidation of the hydrocarbon gas followed by an adiabatic catalytic reforming step and routing the second portion of the hydrocarbon gas feed directly to the adiabatic catalytic reforming step, thus providing a 2-stage synthesis gas production process, which can be described as a separated auto-thermal reforming process, with the POX section resembling the upper part and the adiabatic reforming section resembling the lower part of a conventional ATR reactor, that takes advantage of both the simple non-catalytic partial oxidation (POX) of hydrocarbon gas and the equally simple adiabatic catalytic reforming. The method enables the production of synthesis gas with a higher SN which is more suitable for downstream methanol synthesis. The reformed synthesis gas produced by the method has an SN of about 1.8. Optionally, "about" here means +/- 30% from nominal value.

The first hydrocarbon gas-steam mixture is pre-heated and supplied with the oxidant gas comprising molecular oxygen into the partial oxidation chamber to produce the synthesis gas effluent. The second hydrocarbon gas-steam mixture bypasses the partial oxidation chamber and joins the hot synthesis gas effluent to produce the synthesis gas mixture. The synthesis gas mixture passes over a layer of at least one, for example Ni-based, reforming catalyst in the reforming chamber where the second hydrocarbon gas-steam mixture that is bypassed is adiabatically reformed along with the synthesis gas effluent. The heat from the synthesis gas effluent provides the required heat for the production of the reformed synthesis gas. The synthesis gas mixture reaches chemical equilibrium at the outlet of the adiabatic reforming chamber, because of at least one reforming catalyst.

The first portion of the hydrocarbon gas is mixed with the steam to produce the first hydrocarbon gas-steam mixture. Different amounts of steam may be added to the first portion of the hydrocarbon gas to produce the first hydrocarbon gas-steam mixture. The second portion of the hydrocarbon gas is mixed with the steam to produce the second hydrocarbon gas-steam mixture. Different amounts of steam may be added to the second portion of the hydrocarbon gas to produce the second hydrocarbon gas-steam mixture.

The first hydrocarbon gas-steam mixture is pre-heated and supplied with the oxidant gas comprising molecular oxygen into the partial oxidation chamber to produce the synthesis gas effluent. The first hydrocarbon gas-steam mixture may be pre-heated to different temperatures before supplying with the oxidant gas comprising molecular oxygen into the partial oxidation chamber to produce the synthesis gas effluent. The second hydrocarbon gas-steam mixture is pre-heated before combining with the synthesis gas effluent. The second hydrocarbon gas-steam mixture may be pre-heated to different temperatures before combining with the synthesis gas effluent. In an embodiment, the hydrocarbon gas is a natural gas, biogas, coal gas, shale gas, refinery off gases, waste gases from steel production or other sources.

According to an embodiment, the synthesis gas effluent is produced using a non-catalytic partial oxidation. The non-catalytic partial oxidation is an exothermic reaction where the first hydrocarbon gas-steam mixture reacts with oxygen in the presence of steam as a moderator to produce the synthesis gas effluent. Carbon dioxide may be added as a further moderator.

According to an embodiment, the non-catalytic partial oxidation is performed at a pressure in a range of 25 to 100 Bar and at a temperature that is in a range of 1100 °C to 1450 °C to produce the synthesis gas effluent.

According to an embodiment, the second hydrocarbon gas-steam mixture is pre-heated together with the first hydrocarbon gas-steam mixture. The second hydrocarbon gas-steam mixture is optionally pre-heated together with the first hydrocarbon gas-steam mixture to different temperatures before combining with the synthesis gas effluent.

According to an embodiment, the reforming chamber includes an adiabatic catalytic fixed bed reactor. The adiabatic catalytic fixed bed reactor may include a cylindrical volume filled with at least one reforming catalyst. The synthesis gas mixture may flow downward through the adiabatic catalytic fixed bed reactor to produce the synthesis gas, where a subsequent steam and carbon dioxide (CO₂) reforming takes place.

In an embodiment, the partial oxidation chamber and the adiabatic reforming chamber may be accommodated in a single vessel. The vessel may be lined with a refractory material. According to a second aspect, there is provided a method for producing a methanol from hydrocarbon gas, the method comprising: mixing a first portion of the hydrocarbon gas with steam to produce a first hydrocarbon gas-steam mixture; pre-heating the first hydrocarbon gas-steam mixture before supplying the first hydrocarbon gas-steam mixture with an oxidant gas comprising molecular oxygen into a partial oxidation chamber to produce a synthesis gas effluent; mixing a second portion of the hydrocarbon gas with steam to produce a second hydrocarbon gas-steam mixture, wherein the second hydrocarbon gas-steam mixture is pre-heated before combining with the synthesis gas effluent; combining the synthesis gas effluent with the second hydrocarbon gas-steam mixture to produce a synthesis gas mixture; producing a reformed synthesis gas by adiabatically reforming the synthesis gas mixture over at least one reforming catalyst in an adiabatic reforming chamber; combining the reformed synthesis gas with recovered hydrogen to form a methanol synthesis make-up gas; adding the methanol synthesis make-up gas to a methanol synthesis loop; reacting the methanol synthesis make-up gas in a methanol synthesis unit to produce a crude methanol stream and a purge gas stream; processing the purge gas stream in a hydrogen recovery unit to recover hydrogen from the purge gas stream; and returning the recovered hydrogen to the methanol synthesis loop via the methanol synthesis make-up gas.

The method for producing a methanol from hydrocarbon gas according to present disclosure enables an efficient production of methanol which utilizes the synthesis gas with a stoichiometric number that is higher than that of pure partial oxidation (POX) gas. The reformed synthesis gas produced by the method has an SN of about 1.8, and the methanol synthesis make-up gas has an SN of about 2.0. Optionally, "about" here means +/- 30% from nominal value. The method includes a 2-stage synthesis gas production process that takes advantage of a combined non-catalytic partial oxidation and adiabatic catalytic reforming of hydrocarbon gas for producing a synthesis gas suitable for methanol production. An additional advantage of the method according to the present disclosure is that the method does not require hydrogen recovery from the reformed synthesis gas; instead, the hydrogen is recovered only from the purge gas. The method enables the production of synthesis gas with a higher SN which is more suitable for downstream methanol synthesis.

According to an embodiment, the synthesis gas effluent is produced using a non-catalytic partial oxidation. The non-catalytic partial oxidation is an exothermic reaction where the first hydrocarbon gas-steam mixture reacts with oxygen in the presence of steam as a moderator to produce the synthesis gas effluent. Carbon dioxide may be added as a further moderator.

According to an embodiment, the non-catalytic partial oxidation is performed at a pressure in a range of 25 to 100 Bar and at a temperature in a range of 1100 °C to 1450 °C to produce the synthesis gas effluent. According to an embodiment, the second hydrocarbon gas-steam mixture is pre-heated together with the first hydrocarbon gas-steam mixture. The second hydrocarbon gas-steam mixture is pre-heated together with the first hydrocarbon gas-steam mixture to different temperatures before combining with the synthesis gas effluent.

The second hydrocarbon gas-steam mixture may be pre-heated together with the first hydrocarbon gas-steam mixture up to a point. Moreover, the first hydrocarbon gas-steam mixture and the second hydrocarbon gas-steam mixture may be split and at least one of the first hydrocarbon gas-steam mixture, the second hydrocarbon gas-steam mixture is heated further. There may be one or more heat exchangers/coils to facilitate further heating of the first and the second hydrocarbon gas-steam mixtures.

According to an embodiment, the reforming chamber includes an adiabatic catalytic fixed bed reactor. The adiabatic catalytic fixed bed reactor may include a cylindrical volume filled with at least one reforming catalyst. The synthesis gas mixture may flow downward through the adiabatic catalytic fixed bed reactor to produce the reformed synthesis gas, where a subsequent steam and carbon dioxide (CO₂) reforming takes place.

In an embodiment, the hydrocarbon gas is a natural gas, biogas, coal gas, shale gas, refinery off-gases, waste gases from steel production or other sources.

According to a third aspect, the present disclosure provides an apparatus for producing a synthesis gas for methanol synthesis, the apparatus comprising: a hydrocarbon gas feed supply system comprising a first line for delivering a first portion of the hydrocarbon gas; and a second line for delivering a second portion of the hydrocarbon gas, wherein the first portion of the hydrocarbon gas and the second portion of the hydrocarbon gas are mixed with steam to produce a first hydrocarbon gas-steam mixture and a second hydrocarbon gas-steam mixture respectively; a partial oxidation chamber comprising an inlet for receiving the first hydrocarbon gas-steam mixture and means for mixing the first hydrocarbon gas-steam mixture with an oxidant gas comprising molecular oxygen from an oxidant gas supply system to produce a synthesis gas effluent, wherein the partial oxidation chamber performs partial oxidation on the first hydrocarbon gas-steam mixture using the oxidant gas to produce the synthesis gas effluent; and an outlet for providing the synthesis gas effluent to combine with the second hydrocarbon gas-steam mixture, wherein the synthesis gas effluent is combined with the second hydrocarbon gas-steam mixture to produce a synthesis gas mixture; an adiabatic reforming chamber that is located downstream to the partial oxidation chamber for producing a reformed synthesis gas by adiabatically reforming the synthesis gas mixture received from the partial oxidation chamber, over at least one reforming catalyst in the adiabatic reforming chamber.

The apparatus of producing the synthesis gas for methanol synthesis according to present disclosure enables the production of stoichiometric synthesis gas from the hydrocarbon gas with lower operational costs. The apparatus enables the production of synthesis gas with a higher SN by performing combined non-catalytic partial oxidation (POX) and catalytic reforming of hydrocarbon gas which is more suitable for downstream methanol synthesis with low capital investments. The reformed synthesis gas produced by the apparatus has a SN of about 1.8. Optionally, "about" here means +/- 30% from nominal value.

According to a fourth aspect, there is provided an apparatus for producing a methanol from hydrocarbon gas, the apparatus comprising: a hydrocarbon gas feed supply system comprising a first line for delivering a first portion of the hydrocarbon gas; and a second line for delivering a second portion of the hydrocarbon gas, wherein the first portion of the hydrocarbon gas and the second portion of the hydrocarbon gas are mixed with steam to produce a first hydrocarbon gas-steam mixture and a second hydrocarbon gas-steam mixture respectively; a partial oxidation chamber comprising a first inlet for receiving the first hydrocarbon gas-steam mixture and means for mixing the first hydrocarbon gas-steam mixture with an oxidant gas comprising molecular oxygen from an oxidant gas supply system to produce a synthesis gas effluent, wherein the partial oxidation chamber performs partial oxidation on the first hydrocarbon gas-steam mixture using the oxidant gas to produce the synthesis gas effluent; and a first outlet for providing the synthesis gas effluent to combine with the second hydrocarbon gas-steam mixture, wherein the synthesis gas effluent is combined with the second hydrocarbon gas-steam mixture to produce a synthesis gas mixture; an adiabatic reforming chamber that is located downstream to the partial oxidation chamber for producing a reformed synthesis gas by adiabatically reforming the synthesis gas mixture received from the partial oxidation chamber, over at least one reforming catalyst in the adiabatic reforming chamber; a methanol synthesis unit for converting the reformed synthesis gas into a crude methanol stream and a purge gas stream; a hydrogen recovery unit for recovering hydrogen from the purge gas stream, comprising an inlet for receiving the purge gas stream and outlets for delivering respectively the recovered hydrogen and a tail gas, wherein the recovered hydrogen is combined with the reformed synthesis gas to produce a methanol synthesis make-up gas, wherein the methanol synthesis make-up gas is provided into the methanol synthesis unit to produce the crude methanol stream and the purge gas stream.

The apparatus according to the present disclosure enables an efficient production of methanol which utilizes the reformed synthesis gas with an SN higher than that of pure partial oxidation (POX) gas. The reformed synthesis gas has an SN of about 1.8 and the methanol synthesis make-up gas has an SN of about 2.0. Optionally, "about" here means +/- 30% from nominal value. The apparatus facilitates a 2-stage synthesis gas production process that takes advantage of a combined non-catalytic partial oxidation and adiabatic catalytic reforming of the hydrocarbon gas for producing the reformed synthesis gas for the downstream methanol synthesis. The apparatus enables the production of synthesis gas with a higher SN which is more suitable for downstream methanol synthesis with lower capital investment. The apparatus takes the advantage of a catalytic reformer that provides a simple and straightforward process layout for large-scale production of the synthesis gas. Construction costs of the apparatus are reduced because of its compact design, which can easily be achieved in a single-train.

The synthesis gas mixture prepared by conventional steam reforming of hydrocarbon gas has an excess of hydrogen for the stoichiometry of the methanol synthesis reaction. Therefore, to achieve a more optimum hydrogen/carbon oxides balance, carbon dioxide may be added to the methanol synthesis gas. In the method of the present disclosure, all the hydrogen produced by catalytic reforming of the hydrocarbon gas is used to produce methanol and does not require the added unit operation of adding carbon dioxide. In contradistinction, a main disadvantage of steam reforming is that the size of multi-tubular reactor is quite limited, and the gases obtained are always rich in hydrogen which are unsuitable for methanol synthesis as being over-stoichiometric. Existing systems require external heat transfer devices that results in increased system complexity and potentially high cost. The method of the present disclosure enables the production of stoichiometric synthesis gas by non-catalytic partial oxidation of hydrocarbon gas followed by a simple second adiabatic catalytic reforming step and routing the second portion of the hydrocarbon gas feed directly to the adiabatic catalytic reforming step, thus providing a 2-stage synthesis gas production process that takes advantage of both the simple non-catalytic partial oxidation (POX) of hydrocarbon gas and the equally simple adiabatic catalytic reforming with a low cost operational setup.

In an example embodiment, the method for producing methanol from hydrocarbon gas according to the present disclosure is susceptible to being applied to a small-scale methanol production unit. The methanol synthesis unit includes a simple water-cooled methanol reactor operating at about 60 Bar which is sized for a space velocity of 8500 Normal cubic meter (Nm3/h) per meter cube (m3) of the reforming catalyst. The data in the below table are compared with a plain Partial Oxidation (POX) arrangement for converting the hydrocarbon gas into synthesis gas for methanol production. The stoichiometric number of the synthesis gas at an inlet of the water-cooled methanol reactor is 3.2 in both cases, namely a methanol production capacity 250 metric tons per day (mtpd) of pure methanol. The hydrogen recovery unit optionally reclaims 80% of the hydrogen feed content. The stoichiometric number of 3.2 of the synthesis gas at the water-cooled methanol reactor inlet is achieved by adjusting a recycle ratio in the methanol synthesis loop. With the non-catalytic Partial Oxidation (POX) and the adiabatic catalytic reforming according to the present disclosure, the recycle ratio is 1.83. The recycle ratio is reduced to 0.22 in a plain partial oxidation (POX) arrangement where almost 70% of the unreacted gases are passed to the hydrogen recovery unit. It reduces the loop conversion of CO and CO₂ to methanol significantly.

A table below includes example stream data of methanol production using the method of the present disclosure. In the tables, the following stream label are used:
Stream 01: Natural Gas Feed to POX, Stream 02: Natural Gas Feed to Catalytic Reforming (if applicable), Stream 03: Oxygen, Stream 04: Downstream POX + Catalytic Reforming (if applicable), Stream 05: Downstream Process Condensate Separation, Stream 06: Hydrogen Recycle to Methanol Synthesis, Stream 07: Methanol Synthesis Make-up Feed Stream, Stream 08: Crude Methanol, Stream 09: Purge Gas to Hydrogen Recovery Unit.

| | | | | | | | | | |
|---|---|---|---|---|---|---|---|---|---|
| Stream | 01 | 02 | 03 | 04 | 05 | 06 | 07 | 08 | 09 |
| Temperature [°C] | 300 | 300 | 20 | 992 | 40 | 40 | 40 | 40 | 40 |
| Pressure [bar] | 38 | 38 | 41 | 34.8 | 30.3 | 30.3 | 30.3 | 55.9 | 55.9 |
| Molar Flow [kmol/h] | 365.4 | 84.1 | 209.6 | 1439.3 | 1147.1 | 138.1 | 1285.2 | 389.2 | 238.3 |
| Mass Flow [kg/h] | 6350 | 1460 | 6710 | 18997 | 13707 | 279.2 | 13987 | 11760 | 2227 |
| SN [-] | | | | | 1.79 | | 2.16 | | |
| | mol-% | mol-% | mol-% | mol-% | mol-% | mol-% | mol-% | mol-% | mol-% |
| Hydrogen | 0.00 | 0.00 | 0.00 | 51.56 | 64.69 | 99.98 | 68.48 | 0.59 | 72.45 |
| CO | 0.00 | 0.00 | 0.00 | 20.49 | 25.70 | 0.02 | 22.94 | 0.09 | 5.49 |
| CO₂ | 0.00 | 0.00 | 0.00 | 5.34 | 6.68 | <0.01 | 5.96 | 1.91 | 9.09 |
| CH₄ | 94.00 | 94.00 | 0.00 | 1.98 | 2.48 | <0.01 | 2.22 | 0.35 | 11.39 |
| C₂H₆ | 4.00 | 4.00 | 0.00 | 0.00 | 0.00 | 0.00 | 0.00 | 0.00 | 0.00 |
| C₃H₈ | 1.00 | 1.00 | 0.00 | 0.00 | 0.00 | 0.00 | 0.00 | 0.00 | 0.00 |
| C₄H₁₀ | 0.50 | 0.50 | 0.00 | 0.00 | 0.00 | 0.00 | 0.00 | 0.00 | 0.00 |
| C₅H₁₂ | 0.50 | 0.50 | 0.00 | 0.00 | 0.00 | 0.00 | 0.00 | 0.00 | 0.00 |
| Oxygen | 0.00 | 0.00 | 99.00 | 0.00 | 0.00 | 0.00 | 0.00 | 0.00 | 0.00 |
| Nitrogen | 0.00 | 0.00 | 0.50 | 0.07 | 0.09 | <0.01 | 0.08 | <0.01 | 0.43 |
| Argon | 0.00 | 0.00 | 0.50 | 0.07 | 0.09 | <0.01 | 0.08 | <0.01 | 0.43 |
| Methanol | 0.00 | 0.00 | 0.00 | 0.00 | 0.00 | 0.00 | 0.00 | 84.09 | 0.69 |
| Water | 0.00 | 0.00 | 0.00 | 20.49 | 0.27 | 0.00 | 0.24 | 12.97 | 0.03 |

A next table below includes stream data of a corresponding example of methanol production using plain partial oxidation (POX) for converting the hydrocarbon gas into synthesis gas for methanol production.

| | | | | | | | | | |
|---|---|---|---|---|---|---|---|---|---|
| Stream | 01 | 02 | 03 | 04 | 05 | 06 | 07 | 08 | 09 |
| Temperature [°C] | 300 | 300 | 20 | 1400 | 40 | 40 | 40 | 40 | 40 |
| Pressure [bar] | 38 | 38 | 41 | 34.8 | 30.3 | 30.3 | 30.3 | 56.1 | 56.1 |
| Molar Flow [kmol/h] | 458.4 | 0.0 | 341.7 | 1768.9 | 1363 | 692.7 | 2055.7 | 348.7 | 1038.2 |
| Mass Flow [kg/h] | 7965 | 0 | 10940 | 24429 | 17110 | 1402 | 18512 | 10962 | 7550 |
| SN [-] | | | | | 1.56 | | 2.95 | | |
| | mol-% | mol-% | mol-% | mol-% | mol-% | mol-% | mol-% | mol-% | mol-% |
| Hydrogen | 0.00 | 0.00 | 0.00 | 48.30 | 62.68 | 99.98 | 75.25 | 0.75 | 83.38 |
| CO | 0.00 | 0.00 | 0.00 | 24.10 | 31.28 | 0.02 | 20.75 | 0.19 | 9.75 |
| CO₂ | 0.00 | 0.00 | 0.00 | 4.17 | 5.40 | <0.01 | 3.58 | 1.39 | 5.68 |
| CH₄ | 94.0 0 | 94.0 0 | 0.00 | 0.10 | 0.13 | <0.0 1 | 0.08 | <0.01 | 0.17 |
| C₂H₆ | 4.00 | 4.00 | 0.00 | 0.00 | 0.00 | 0.00 | 0.00 | 0.00 | 0.00 |
| C₃H₈ | 1.00 | 1.00 | 0.00 | 0.00 | 0.00 | 0.00 | 0.00 | 0.00 | 0.00 |
| C₄H₁₀ | 0.50 | 0.50 | 0.00 | 0.00 | 0.00 | 0.00 | 0.00 | 0.00 | 0.00 |
| C₅H₁₂ | 0.50 | 0.50 | 0.00 | 0.00 | 0.00 | 0.00 | 0.00 | 0.00 | 0.00 |
| Oxygen | 0.00 | 0.00 | 99.00 | 0.00 | 0.00 | 0.00 | 0.00 | 0.00 | 0.00 |
| Nitrogen | 0.00 | 0.00 | 0.50 | 0.10 | 0.12 | <0.01 | 0.08 | <0.01 | 0.16 |
| Argon | 0.00 | 0.00 | 0.50 | 0.10 | 0.12 | <0.01 | 0.08 | <0.01 | 0.16 |
| Methanol | 0.00 | 0.00 | 0.00 | 0.00 | 0.00 | 0.00 | 0.00 | 93.83 | 0.70 |
| Water | 0.00 | 0.00 | 0.00 | 23.13 | 0.27 | 0.00 | 0.18 | 3.84 | <0.01 |

A next table below includes the process key indicators of the present disclosed example according to the method of the present disclosure in relation to the plain partial oxidation example.

| Process Key Indicator | Percentage of Novel method versus plain POX |
|---|---|
| Hydrocarbon gas feed flow | 80% |
| Oxygen flow | 61% |
| Make-up gas flow | 63% |
| Feed to Hydrogen Recovery unit flow | 23% |
| Compressor power (Make-up + recycle) | 72% |
| Methanol Reactor Catalyst amount | 144% |
| Carbon feedstock conversion to methanol (percentage) | 132% |

The above table shows that the methanol synthesis according to the method of the present disclosure is much more efficient than plain Partial Oxidation (POX) of the synthesis gas for methanol production. Thus, the method of the present disclosure for methanol synthesis is capable of greatly reducing operational costs and investment cost.

### DETAILED DESCRIPTION OF THE DRAWINGS

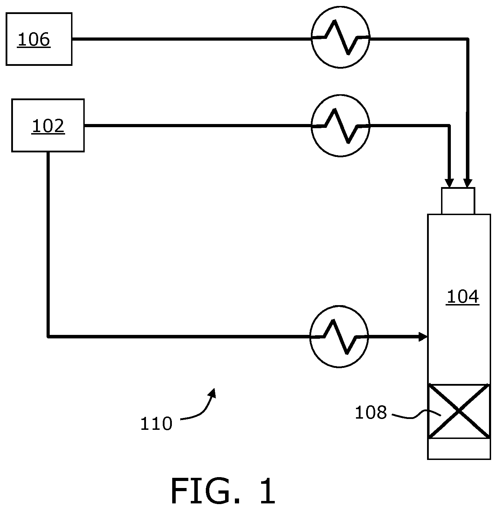

**FIG. 1** is a schematic illustration of an apparatus **110** for producing a synthesis gas for methanol synthesis according to an embodiment of the present disclosure. The apparatus **110** includes a hydrocarbon gas feed supply system **102,** a partial oxidation chamber **104,** an oxidant gas supply system **106** and an adiabatic reforming chamber **108.** The hydrocarbon gas feed supply system **102** includes a first line for delivering a first portion of the hydrocarbon gas and a second line for delivering a second portion of the hydrocarbon gas. The first portion of the hydrocarbon gas is mixed with steam to produce a first hydrocarbon gas-steam mixture. The first hydrocarbon gas-steam mixture is pre-heated before supplying to the partial oxidation chamber **104.** The second portion of the hydrocarbon gas is mixed with steam to produce a second hydrocarbon gas-steam mixture. The partial oxidation chamber **104** includes an inlet for receiving the first hydrocarbon gas-steam mixture which is mixed with an oxidant gas comprising molecular oxygen from the oxidant gas supply system **106** and converted under partial oxidation conditions to produce a synthesis gas effluent. A partial oxidation is performed on the first hydrocarbon gas-steam mixture using the oxidant gas comprising molecular oxygen to produce the synthesis gas effluent in the partial oxidation chamber **104.** The synthesis gas effluent is provided from the partial oxidation chamber **104** through an outlet to combine with the second hydrocarbon gas-steam mixture to produce a synthesis gas mixture. The second hydrocarbon gas-steam mixture is also pre-heated before combining with the synthesis gas effluent. The adiabatic reforming chamber **108** is located downstream to the partial oxidation chamber **104** for producing a reformed synthesis gas by adiabatically reforming the synthesis gas mixture received from the partial oxidation chamber **104** over at least one reforming catalyst in the adiabatic reforming chamber **108.**

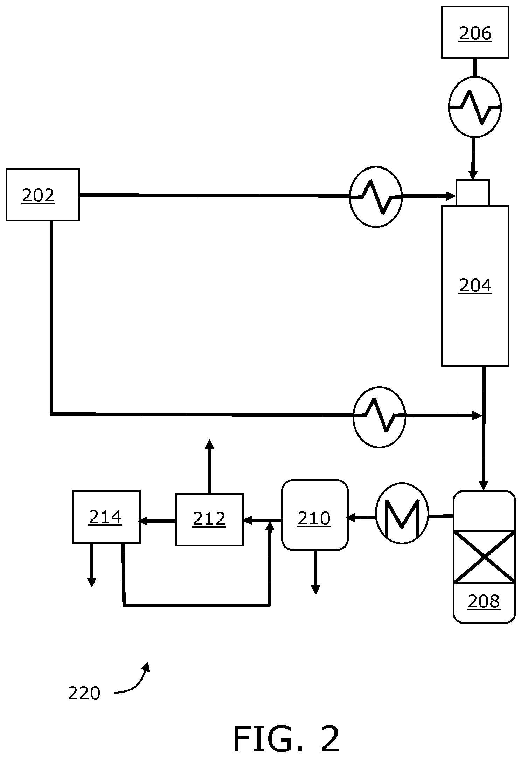

**FIG. 2** is a schematic illustration of an apparatus **220** for producing a methanol from hydrocarbon gas according to an embodiment of the present disclosure. The apparatus **220** includes a hydrocarbon gas feed supply system **202,** a partial oxidation chamber **204,** an oxidant gas supply system **206,** an adiabatic reforming chamber **208,** a condensate collection chamber **210,** a methanol synthesis unit **212** and a hydrogen recovery unit **214.** The hydrocarbon gas feed supply system **202** includes a first line for delivering a first portion of the hydrocarbon gas and a second line for delivering a second portion of the hydrocarbon gas. The first portion of the hydrocarbon gas is mixed with steam to produce a first hydrocarbon gas-steam mixture. The first hydrocarbon gas-steam mixture is pre-heated before supplying to the partial oxidation chamber **204.** The second portion of the hydrocarbon gas is mixed with steam to produce a second hydrocarbon gas-steam mixture. The partial oxidation chamber **204** includes a first inlet for receiving the first hydrocarbon gas-steam mixture with an oxidant gas comprising molecular oxygen from the oxidant gas supply system **206** and converted under partial oxidation conditions to produce a synthesis gas effluent. A partial oxidation is performed on the first hydrocarbon gas-steam mixture using the oxidant gas comprising molecular oxygen to produce the synthesis gas effluent in the partial oxidation chamber **204.** The synthesis gas effluent is provided from the partial oxidation chamber **204** through a first outlet to combine with the second hydrocarbon gas-steam mixture to produce a synthesis gas mixture. The second hydrocarbon gas-steam mixture is also pre-heated before combining with the synthesis gas effluent. The adiabatic reforming chamber **208** is located downstream to the partial oxidation chamber **204** for producing a reformed synthesis gas by adiabatically reforming the synthesis gas mixture received from the partial oxidation chamber **204** over at least one reforming catalyst in the adiabatic reforming chamber **208.** A process condensate which is formed after the reforming process in the adiabatic reforming chamber **208** is optionally removed by collecting it in a condensate collection chamber **210.** The reformed synthesis gas is converted into a crude methanol stream and a purge gas stream in the methanol synthesis unit **212.** The hydrogen recovery unit **214** recovers hydrogen from the purge gas stream. The hydrogen recovery unit **214** includes an inlet for receiving the purge gas stream and two outlets for delivering respectively the recovered hydrogen and a tail gas. The recovered hydrogen is combined with the reformed synthesis gas to produce a methanol synthesis make-up gas. The methanol synthesis make-up gas is directed into the methanol synthesis unit **212** to produce the crude methanol stream and the purge gas stream. The tail gas is removed from the hydrogen recovery unit **214.** The methanol synthesis make-up gas is produced by combining the reformed synthesis gas with the recovered hydrogen. The reformed synthesis gas has an SN of about 1.8 and the methanol synthesis make-up gas has an SN of about 2.0. Optionally, "about" here means +/- 30% from nominal value.

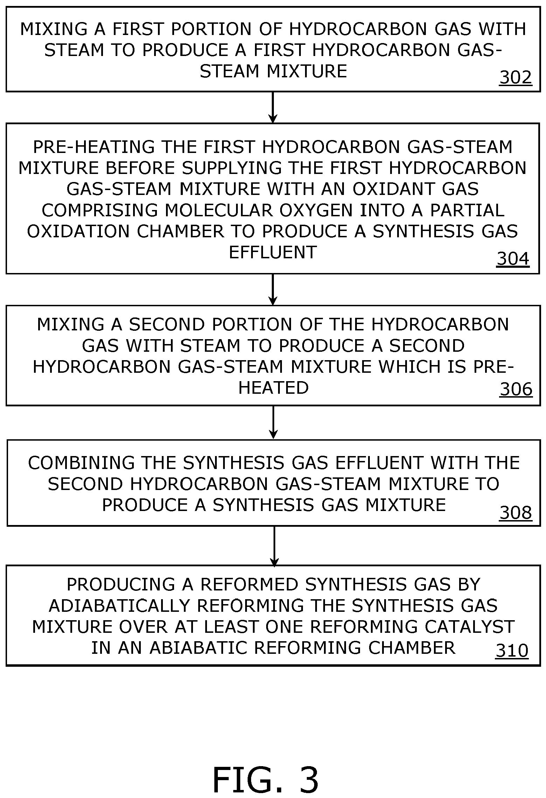

**FIG. 3** is a flowchart illustrating a method for producing a synthesis gas for methanol synthesis according to an embodiment of the present disclosure. At a step **302,** a first portion of hydrocarbon gas is mixed with steam to produce a first hydrocarbon gas-steam mixture. At a step **304,** the first hydrocarbon gas-steam mixture is pre-heated before supplying the first hydrocarbon gas-steam mixture with an oxidant gas comprising molecular oxygen into a partial oxidation chamber to produce a synthesis gas effluent. At a step **306,** a second portion of the hydrocarbon gas is mixed with steam to produce a second hydrocarbon gas-steam mixture which is pre-heated before combining with the synthesis gas effluent. At a step **308,** the synthesis gas effluent is combined with the second hydrocarbon gas-steam mixture to produce a synthesis gas mixture. At a step **310,** a reformed synthesis gas is produced by adiabatically reforming the synthesis gas mixture over at least one reforming catalyst in an adiabatic reforming chamber.

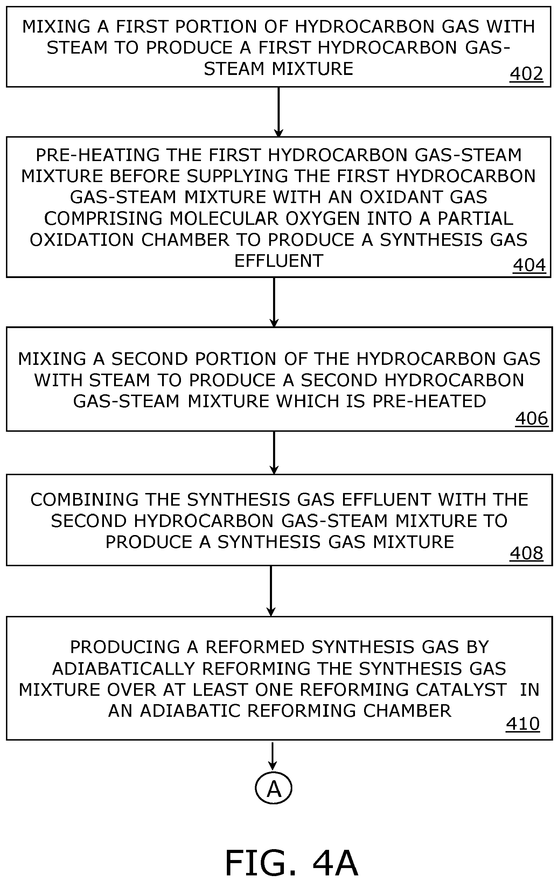

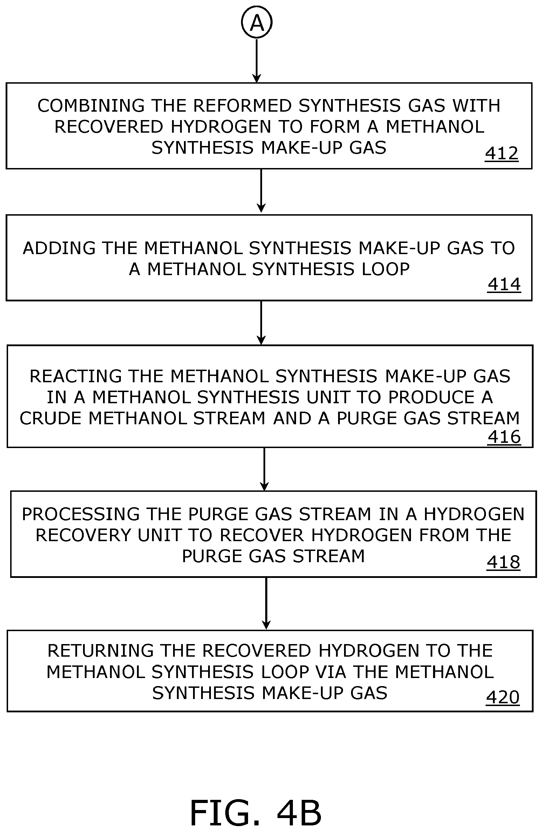

**FIGS. 4A** **and** **4B** are flowcharts illustrating a method for producing a methanol from hydrocarbon gas according to an embodiment of the present disclosure. At a step **402,** a first portion of hydrocarbon gas is mixed with steam to produce a first hydrocarbon gas-steam mixture. At a step **404,** the first hydrocarbon gas-steam mixture is pre-heated before supplying the first hydrocarbon gas-steam mixture with an oxidant gas comprising molecular oxygen into a partial oxidation chamber to produce a synthesis gas effluent. At a step **406,** a second portion of the hydrocarbon gas is mixed with steam to produce a second hydrocarbon gas-steam mixture which is pre-heated before combining with the synthesis gas effluent. At a step **408,** the synthesis gas effluent is combined with the second hydrocarbon gas-steam mixture to produce a synthesis gas mixture. At a step **410,** a reformed synthesis gas is produced by adiabatically reforming the synthesis gas mixture over at least one reforming catalyst in an adiabatic reforming chamber. At a step **412,** the reformed synthesis gas is combined with recovered hydrogen to form a methanol synthesis make-up gas. At a step **414,** the methanol synthesis make-up gas is added to a methanol synthesis loop. At a step **416,** the reformed synthesis gas is reacted in a methanol synthesis unit to produce a crude methanol stream and a purge gas stream. At a step **418,** the purge gas stream is processed in a hydrogen recovery unit to recover hydrogen from the purge gas stream. At a step **420,** the recovered hydrogen is returned to the methanol synthesis loop via the methanol synthesis make-up gas. The methanol synthesis make-up gas is produced by combining the reformed synthesis gas with the recovered hydrogen. The reformed synthesis gas has an SN of about 1.8 and the methanol synthesis make-up gas has an SN of about 2.0. Optionally, "about" here means +/- 30% from nominal value.

Modifications to embodiments of the present disclosure described in the foregoing are possible without departing from the scope of the present disclosure as defined by the accompanying claims. Expressions such as "including", "comprising", "incorporating", "have", "is" used to describe, and claim the present disclosure are intended to be construed in a non-exclusive manner, namely allowing for items, components or elements not explicitly described also to be present. Reference to the singular is also to be construed to relate to the plural.

### LIST OF REFERENCE NUMERALS

- 110, 220: apparatus
- 102, 202: hydrocarbon gas feed supply system
- 104, 204: partial oxidation chamber
- 106, 206: oxidant gas supply system
- 108, 208: adiabatic reforming chamber
- 210: condensate collection chamber
- 212: methanol synthesis unit
- 214: hydrogen recovery unit

## Claims

1. A method for producing a synthesis gas for methanol synthesis, the method comprising:
mixing a first portion of hydrocarbon gas with steam to produce a first hydrocarbon gas-steam mixture;
pre-heating the first hydrocarbon gas-steam mixture before supplying the first hydrocarbon gas-steam mixture with an oxidant gas comprising molecular oxygen into a partial oxidation chamber to produce a synthesis gas effluent;
mixing a second portion of the hydrocarbon gas with steam to produce a second hydrocarbon gas-steam mixture, wherein the second hydrocarbon gas-steam mixture is pre-heated before combining with the synthesis gas effluent;
combining the synthesis gas effluent with the second hydrocarbon gas-steam mixture to produce a synthesis gas mixture; and
producing a reformed synthesis gas by adiabatically reforming the synthesis gas mixture over at least one reforming catalyst in an adiabatic reforming chamber (108, 208).

2. The method of claim 1, wherein the method includes producing the synthesis gas effluent using a non-catalytic partial oxidation.

3. The method of claim 2, wherein the method includes performing the non-catalytic partial oxidation at a pressure in a range of 25 to 100 Bar and at a temperature that is in the range of 1100 to 1450 °C to produce the synthesis gas effluent.

4. The method of any one of claims 1 to 3, wherein the method includes pre-heating the second hydrocarbon gas-steam mixture together with the first hydrocarbon gas-steam mixture.

5. The method of any one of the preceding claims, wherein the method includes arranging for the reforming chamber (108, 208) to comprise an adiabatic catalytic fixed bed reactor.

6. A method for producing methanol from hydrocarbon gas, the method comprising:
mixing a first portion of the hydrocarbon gas with steam to produce a first hydrocarbon gas-steam mixture;
pre-heating the first hydrocarbon gas-steam mixture before supplying the first hydrocarbon gas-steam mixture with an oxidant gas comprising molecular oxygen into a partial oxidation chamber to produce a synthesis gas effluent;
mixing a second portion of the hydrocarbon gas with steam to produce a second hydrocarbon gas-steam mixture, wherein the second hydrocarbon gas-steam mixture is pre-heated before combining with the synthesis gas effluent;
combining the synthesis gas effluent with the second hydrocarbon gas-steam mixture to produce a synthesis gas mixture;
producing a reformed synthesis gas by adiabatically reforming the synthesis gas mixture over at least one reforming catalyst in an adiabatic reforming chamber (108, 208);
combining the reformed synthesis gas with recovered hydrogen to form a methanol synthesis make-up gas;
adding the methanol synthesis make-up gas to a methanol synthesis loop;
reacting the methanol synthesis make-up gas in a methanol synthesis unit to produce a crude methanol stream and a purge gas stream;
processing the purge gas stream in a hydrogen recovery unit to recover hydrogen from the purge gas stream; and
returning the recovered hydrogen to the methanol synthesis loop via the methanol synthesis make-up gas.

7. The method of claim 6, wherein the method includes producing the synthesis gas effluent using a non-catalytic partial oxidation.

8. The method of claim 7, wherein the method includes performing the non-catalytic partial oxidation at a pressure in a range of 25 to 100 Bar and at a temperature that is in the range of 1100 to 1450 °C to produce the synthesis gas effluent.

9. The method of any one of the claims 6 to 8, wherein the method includes arranging for the reforming chamber (108, 208) to comprise an adiabatic catalytic fixed bed reactor.

10. An apparatus (110, 220) for producing a synthesis gas for methanol synthesis, the apparatus (110, 220) comprising:
a hydrocarbon gas feed supply system (102, 202) comprising
a first line for delivering a first portion of the hydrocarbon gas; and
a second line for delivering a second portion of the hydrocarbon gas,
wherein the first portion of the hydrocarbon gas and the second portion of the hydrocarbon gas are mixed with steam to produce a first hydrocarbon gas-steam mixture and a second hydrocarbon gas-steam mixture respectively;
a partial oxidation chamber (104, 204) comprising
an inlet for receiving the first hydrocarbon gas-steam mixture and means for mixing the first hydrocarbon gas-steam mixture with an oxidant gas comprising molecular oxygen from an oxidant gas supply system (106, 206) to produce a synthesis gas effluent, wherein the partial oxidation chamber (104, 204) performs partial oxidation on the first hydrocarbon gas-steam mixture using the oxidant gas comprising molecular oxygen to produce the synthesis gas effluent; and
an outlet for providing the synthesis gas effluent to combine with the second hydrocarbon gas-steam mixture, wherein the synthesis gas effluent is combined with the second hydrocarbon gas-steam mixture to produce a synthesis gas mixture;
an adiabatic reforming chamber (108, 208) that is located downstream to the partial oxidation chamber (104, 204) for producing a reformed synthesis gas by adiabatically reforming the synthesis gas mixture, over at least one reforming catalyst in the adiabatic reforming chamber (108, 208).

11. An apparatus (110, 220) for producing methanol from hydrocarbon gas, the apparatus (110, 220) comprising:
a hydrocarbon gas feed supply system (102, 202) comprising
a first line for delivering a first portion of the hydrocarbon gas; and
a second line for delivering a second portion of the hydrocarbon gas,
wherein the first portion of the hydrocarbon gas and the second portion of the hydrocarbon gas are mixed with steam to produce a first hydrocarbon gas-steam mixture and a second hydrocarbon gas-steam mixture, respectively;
a partial oxidation chamber (104, 204) comprising
a first inlet for receiving the first hydrocarbon gas-steam mixture and means for mixing the first hydrocarbon gas-steam mixture with an oxidant gas comprising molecular oxygen from an oxidant gas supply system (106, 206) to produce a synthesis gas effluent, wherein the partial oxidation chamber (104, 204) performs partial oxidation on the first hydrocarbon gas-steam mixture using the oxidant gas comprising molecular oxygen to produce the synthesis gas effluent; and
a first outlet for providing the synthesis gas effluent to combine with the second hydrocarbon gas-steam mixture, wherein the synthesis gas effluent is combined with the second hydrocarbon gas-steam mixture to produce a synthesis gas mixture;
an adiabatic reforming chamber (108, 208) that is located downstream to the partial oxidation chamber (104,204) for producing a reformed synthesis gas by adiabatically reforming the synthesis gas mixture, received from the partial oxidation chamber (104, 204), over at least one reforming catalyst in the adiabatic reforming chamber (108, 208);
a methanol synthesis unit (212) for converting the synthesis gas into a crude methanol stream and a purge gas stream; and
a hydrogen recovery unit (214) for recovering hydrogen from the purge gas stream, comprising an inlet for receiving the purge gas stream and two outlets for delivering respectively the recovered hydrogen and a tail gas, wherein the recovered hydrogen is combined with the reformed synthesis gas to produce a methanol synthesis make-up gas, wherein the methanol synthesis make-up gas is provided into the methanol synthesis unit (212) to produce the crude methanol stream and the purge gas stream.
